# EUROPÄISCHE PATENTANMELDUNG

(11) **EP 1 285 640 A2**
(43) Veröffentlichungstag der Anmeldung: **26.02.2003**
(21) Anmeldenummer: 02016775.5
(22) Anmeldetag: 26.07.2002
(51) Int. Cl.: A61F 2/60, A61F 2/68

(54) **Sensoreinrichtung, insbesondere für eine Prothese, Prothese mit dieser Sensoreinrichtung**

(30) Priorität: 10.08.2001 DE 10139333
(71) Anmelder: BIEDERMANN MOTECH GmbH, 78054 VS-Schwenningen (DE)
(72) Erfinder: Biedermann, Lutz, 78048 VS-Villingen (DE); Matthis, Wilfried, 79367 Weisweil (DE)
(74) Vertreter: Prüfer, Lutz H., Dipl.-Phys.

(57) **Zusammenfassung**

Es wird eine Sensoreinrichtung (15) für eine Prothese, insbesondere für eine Beinprothese, bereitgestellt zum Messen von auf die Prothese wirkenden Kräften, mit einem ringförmig geschlossen ausgebildeten Außenkörper (16) und einem zwei gegenüberliegende Innenseiten (17a, 17b) des Außenkörpers verbindendem Innenkörper (21, 22) mit einem Sensorelement (31) zur Messung der in Richtung der Verbindungsachse wirkenden Kraft. Der Außenkörper verbiegt sich bei Einwirkung eines Biegemoments, während der Innenkörper (21) nur die axiale in Richtung seiner Verbindungsachse wirkende Kraft sieht. Dadurch ist eine Messung der axialen Kraft ohne Beeinflussung von Biegemomenten möglich. Ferner ist die Sensoreinrichtung in der Lage Lasten zu übertragen.

## Beschreibung

Die Erfindung betrifft eine Sensoreinrichtung, insbesondere für eine Prothese und bevorzugt für eine Beinprothese, und eine Prothese mit einer derartigen Sensoreinrichtung.

Bei mechanischen Prothesensystemen muß sichergestellt sein, daß auch unter extremen Lastsituationen bzw. bei aktivem Gehen kein Prothesenbauteil durch zyklische Überlastung versagt. Für den Prothesenkonstrukteur ergibt sich dadurch eine Schwierigkeit dahingehend, daß das beim Design der Prothese zu berücksichtigende Spektrum der Lasten sehr breit ist und Teillastkomponenten nur ungenügend bekannt sind. Da aber Baulänge, Gewicht und Volumen kritische Designfaktoren sind, sind die meisten Bauteile der Prothese in Leichtbauweise ausgelegt, d.h. die meisten Prothesenteile, inbesondere der Prothesencarrier sind nur für eine bestimmte Anzahl von Betriebszyklen ausgelegt.

Bei elektronisch angesteuerten Prothesen ergeben sich weitere Probleme, da Gewicht durch Batteriekomponenten und aktive Bauteile zur Bewegungssteuerung hinzukommen, wodurch die Anforderungen an die Werkstoffausnutzung weiter erhöht sind. Zusätzlich ist eine Sensoreinheit zur Messung der Bodenreaktionskraft für die Ganganalyse erforderlich. Ein derartiger Sensor beinhaltet in den meisten Fällen Axialkraft- und Biegemomentmessung. Dabei gibt es ein technisches Problem dahingehend, daß die Dehnungen durch Axialkraft, die maximal etwa 1500 N beträgt, etwa um den Faktor 5 bis 10 niedriger liegen, als die Dehnungen durch Biegemomente, die bei etwa 150 Nm liegen. Da jeder Dehnungs-Sensor, wie z.B. ein DMS-,Hall oder Kapazitäten-Sensor, aber beide Dehnungen als Kombination sieht, ist eine optimale mechanische Konstruktion zur Lasttrennung nötig. Dies wird durch die geringen Axialkräfte sowie durch zusätliche Belastungskomponenten wie z.B. seitliche Biegung- und Torsionsmomente erschwert. Weiterhin ist eine hohe Dehnung im Hinblick auf die Axialkraft erforderlich, um einen stabilen Nullpunkt zu erzielen.

Aus der WO 00/38 599 ist eine elektronisch angesteuerte Beinprothese mit einem Oberschenkelteil und einem Unterschenkelteil und einem die beiden verbindenden Kniegelenk bekannt, bei der Kraftsensoren zur Messung der auf die Prothese wirkenden Gesamtkraft in dem Fußsohlenbereich vorgesehen sind. Ferner sind bei dieser Prothese Kraftsensoren zur Messung der Gesamtkraft bzw. auch der Biegekraft im Schienbeinteil vorgesehen. Die Messung der Gesamtkraft ist jedoch nicht unabhängig von Einflüssen von Biegemonenten.

Es ist Aufgabe der Erfindung, eine Sensoreinrichtung, insbesondere für eine Prothese und bevorzugt für eine Beinprothese, und eine derartige Prothese mit einer solchen Sensoreinrichtung bereitzustellen, bei die zu erwartenden Belastungen sicher übertragen werden und ein genaues Messen möglich ist.

Die Aufgabe wird gelöst durch eine Sensoreinrichtung nach Patentanspruch 1 oder 14 bzw. eine Prothese mit einer derartigen Sensoreinrichtung gemäß Patentanspruch 13.

Weiterbildungen der Erfindung sind in den Unteransprüchen angegeben.

Die erfindungsgemäße Sensoreinrichtung weist den Vorteil auf, dass Belastungen auf die Prothese durch die Sensoreinrichtung sicher übertragen werden. Ferner besteht der Vorteil der Lasttrennung von Axialbelastung und Biegebelastung. Durch die Lasttrennung lassen sich bei einer Beinprothese die Gangphasen Bodenauftritt, Abrollen und Abheben genau bestimmen. Außerdem ist ein stabiler Nullpunkt unabhängig von der Umgebung, d. h. von Temperaturschwankungen oder Kräften beim Zusammenbau wie z. B. durch Schrauben ausgeübt, gegeben. Somit besteht keine Sensordrift.

Weitere Merkmale und Zweckmäßigkeiten der Erfindung ergeben sich aus der Beschreibung von Ausführungsbeispielen anhand der Figuren.

Von den Figuren zeigen:
- Fig. 1(a)-(b): eine Beinprothese mit der erfindungsgemäßen Sensoreinrichtung in unterschiedlichen Gangpositionen;
- Fig. 2: eine seitlich perspektivische Ansicht einer ersten Ausführungsform einer Sensoreinrichtung in einem Schienbeinteil einer Beinprothese zusammen mit einem Koordinatensystem;
- Fig.3: eine vergrößerte seitlich perspektivische Ansicht der Sensoreinrichtung;
- Fig. 4: eine vergrößerte perspektivische Darstellung eines Innenteils der Sensoreinrichtung von Fig. 3;
- Fig. 5(a)-(b): eine schematische Darstellung der Verformung der Sensoreinrichtung unter verschiedenen Belastungen;
- Fig. 6: eine schematische Darstellung einer weiteren Ausführungsform der Sensoreinrichtung; und
- Fig. 7: eine schematische Darstellung einer weiteren Ausführungsform der Beinprothese.

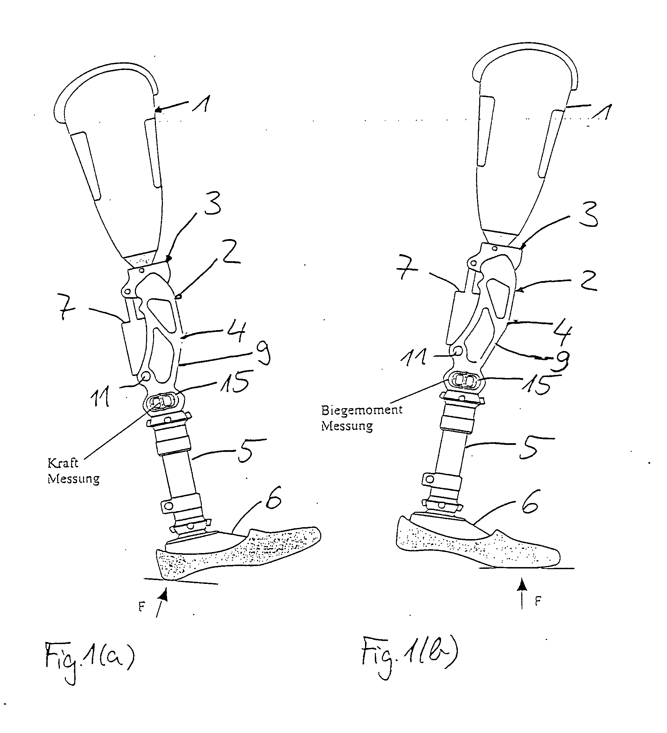

Wie aus den Figuren 1(a) und 1(b) ersichtlich ist, umfasst eine Beinprothese in bekannter Weise ein Oberschenkelteil 1, ein Unterschenkelteil 2 und ein die beiden verbindendes Kniegelenk 3. Das Unterschenkelteil 2 weist ein Schienbeinteil 4 mit einem Unterschenkelrohr 5 und ein mit diesem verbundenes Fußteil 6 auf. Das Fußteil 6 weist in bekannter Weise eine in den Figuren nicht dargestellt Blattfeder zum Ermöglichen eines federnden Auftrittes auf. Das Oberschenkelteil 1 ist zum Verbinden mit einem Beinstumpf ausgebildet. Das künstliche Kniegelenk 3 beinhaltet ein Dämpfungselement in Form einer Kolben-Zylindereinrichtung 7.

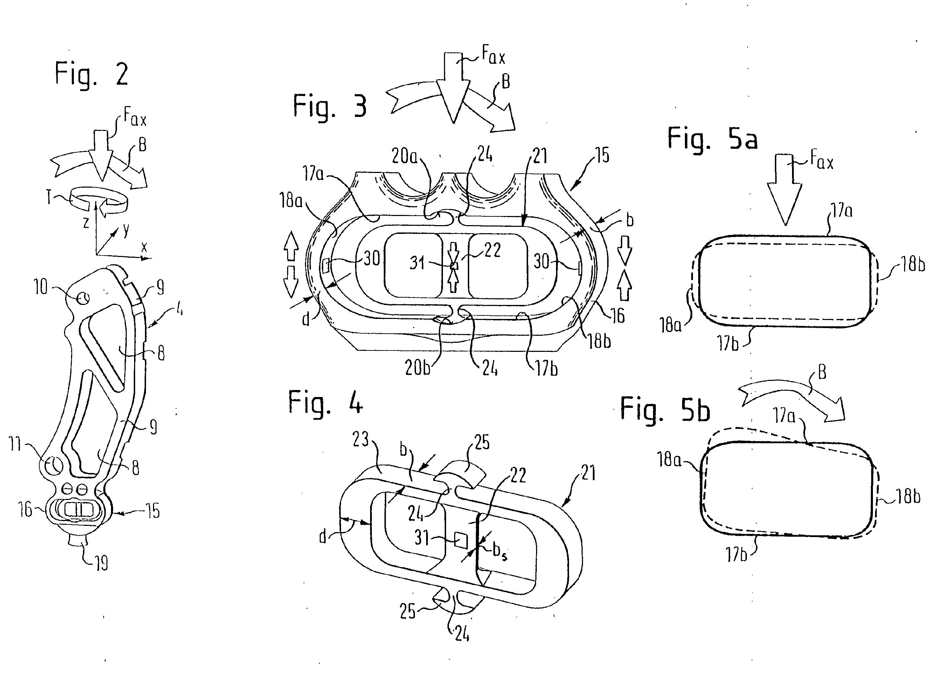

Wie aus den Figuren 1(a), 1(b) und 2 ersichtlich ist, ist das Schienbeinteil 4 aus zwei parallel zueinander verlaufenden, mit Aussparungen 8 zur Gewichtsersparnis versehenen länglichen Seitenteilen 9 ausgebildet, die an ihrem dem Oberschenkelteil 1 zugewandten Ende Bohrungen 10 zur Aufnahme einer Schwenkachse des künstlichen Kniegelenks 3 aufweisen und in ihrem dem Oberschenkelteil 1 abgewandten Ende korrespondierende Öffnungen 11 zum Verbinden mit der Kolben-Zylindereinrichtung 7 aufweisen. Die Seitenteile 9 sind an ihrem dem Oberschenkelteil 1 abgewandten Ende über eine Sensoreinrichtung 15 verbunden. In dem gezeigten Ausführungsbeispiel sind die Seitenteile 9 und die Sensoreinrichtung einstückig ausgebildet.

In Fig. 2 ist ferner ein rechtwinkliges Koordinatensystem für die Prothese und die auftretenden Kräfte dargestellt. Die z-Achse verläuft dabei in Richtung einer mittig durch die Schwenkachse des künstlichen Kniegelenks hindurchgehende Verbindungsachse zwischen Oberschenkelteil 1 und Schienbeinteil 4 bei gestreckter Stellung der Prothese. Die x-Achse und die z-Achse definieren eine Schwenkebene des künstlichen Kniegelenks.

Die Sensoreinrichtung 15 besteht, wie aus den Figuren 2 bis 4 ersichtlich ist, aus einem im wesentlichen ovalen ringförmigen Außenkörper 16 mit einer Breite b in y-Richtung, also senkrecht zur Schwenkebene, die in etwa dem Abstand der Seitenteile 9 des Schienbeinteils entspricht. Die Längsseiten 17a, 17b des ovalen Rings sind senkrecht zur z-Achse in x-Richtung orientiert. Die Seitenteile 9 des Schienbeinteils 4 erstrecken sich an gegenüberliegenden Rändern einer der Längsseiten 17a des ovalen Ringes in Richtung des Oberschenkelteils. Die kurzen Seitenabschnitte des ovalen Ringes bilden Stege 18a, 18b mit einer vorbestimmten Dicke d. An den dem ovalen Innenraum des Außenkörpers 16 zugewandten Innenseiten der Längsseiten 17a, 17b sind mittig zwei einander gegenüberliegende und sich über die gesamte Breite des Rings erstreckende rillenförmige Ausnehmungen 20a, 20b vorgesehen, die zur Aufnahme eines weiter unten beschriebenen Innenkörpers dienen. An seiner den Seitenteilen 9 des Schienbeinteils 4 abgewandten Längsseite 17b weist die Sensoreinrichtung einen sich konisch verjüngenden Abschnitt mit einem Ansatz 19 zum Verbinden mit dem Unterschenkelrohr 5 auf.

Der Außenkörper 16 ist aus einem Material gebildet, welches leicht ist und in der gezeigten Ringform eine gewünschte Elastizität unter Einwirkung von in Richtung der z-Achse wirkenden axialen Kräften und in der Schwenkebene wirkenden Biegekräften aufweist. Z.B. ist der Außenkörper aus Aluminium gebildet. Die Dicke d des Rings ist so gewählt, daß die für eine Messung der Kräfte erwünschten Verformungen unter Belastung auftreten.

An den dem Innenraum zugewandten Seiten der Stege 18a, 18b sind in diesem Ausführungsbeispiel an einander gegenüberliegenden Positionen Sensorelemente 30 vorgesehen zur Erfassung eines in Fig. 2 durch den geschwungenen Pfeil dargestellten Biegemomentes B in Schwenkrichtung der Prothese. Die Sensorelemente sind beispielsweise als Dehnungsmeßstreifen (DMS)-Streifen ausgebildet.

Wie aus den Figuren 3 und 4 ersichtlich ist, weist die Sensoreinrichtung ferner einen im Inneren des ovalen Rings des Außenkörpers 16 angeordneten Innenkörper 21 zur Erfassung der axialen, in z-Richtung wirkenden Kraft Fₐₓ, die durch den geraden Pfeil in Fig. 2 dargestellt ist, auf. Der Innenkörper 21 besteht aus einem stegförmig ausgebildeten Sensorteil 22 mit im wesentlichen rechteckigem Querschnitt an dem ein Sensorelement 31, beispielsweise ein DMS-Streifen angebracht ist. In dem gezeigten Ausführungsbeispiel bildet das Sensorteil 22 einen mittig zwischen den Innenseiten der Längsseiten eines im wesentlichen rechteckigen Rings 23 vorgesehenen Verbindungssteg. Die Außen- und Innenkanten des Rechtecks sind abgerundet. Die Breite b des Rings 23 in y-Richtung ist deutlich kleiner als die des Außenkörpers 16. Wie insbesondere aus Fig. 4 ersichtlich ist, ist die Breite bₛ des Sensorteils 22 in y-Richtung kleiner als die des Rings 23. Vorteilhafterweise ist die Breite bₛ des Sensorteils 22 etwa ein Viertel bis ein Drittel der Breite b des Rings 23. Dadurch weist das Sensorteil 22 einen kleinen Querschnitt auf, der eine optimale Messung der axialen Kraft Fₐₓ erlaubt. Die Dicke d des Rings an den kurzen Seiten ist größer als die an den langen Seiten wodurch die kurzen Seiten Ausgleichskörper zur Biegeentlastung des Sensorteils 22 bilden. Die Dicke d, der kurzen Seiten, die Breite des Ringes und seine Durchmesser sind derart gewählt, daß sich durch die so gebildeten Massekörper eine dem zu erwartenden auftretenden Biegemoment entsprechende gewünschte Biegeentlastung ergibt.

Der Ring 23 weist ferner an den Außenseiten der Längsseiten an der dem Sensorteil 22 abgewandten Stellen sich über die Breite b des Ringes 23 nach außen erstreckenden stegförmige Ansätze 24 auf, an deren freiem Ende ein zylindersegmentförmiger Abschnitt 25 angeformt ist, dessen Abmessungen so sind, daß er in die rillenförmigen Ausnehmungen 20a, 20b des Außenteils paßt. Die Dicke der Stege 24 in x-Richtung ist kleiner als die des Sensorteils 22 um eine kleine Angriffsfläche für Biegemomente zu bieten und damit die Übertragung von Biegekräften in der z-x-Ebene möglichst gering zu halten. Idealerweise ist die Dicke der stegförmigen Ansätze 24 so bemessen, daß sich eine punktweise bzw. momentenfreie oder momentenarme Verbindung zu dem Außenkörper 16 ergibt. Eine solche Verbindung verhindert bzw. minimiert Nebeneinflüsse auf die Axialkraft durch überlagerte Biegemomente. Ferner ist die Länge der Stege 24 ist so gewählt, daß der aus Ring 23 und Sensorteil 22 bestehende Innenkörper genau in den Außenkörper 16 einfügbar ist. Der Innenkörper ist aus demselben Material gebildet wie der Außenkörper um temperaturbedingte Spannungen möglichst zu vermeiden. In zusammengesetztem Zustand ist der Innenkörper in die rillenförmigen Ausnehmungen 20a, 20b des Außenkörpers eingesetzt und an dieser Stelle mit dem fest verbunden, z.B. verklebt oder verschweißt. Der Sensorteil 22 ist somit in z-Richtung der Prothese orientiert und die Längsseiten des Außenkörpers 16 und des Rings 23 des Innenkörpers sind parallel.

Im Betrieb der Prothese, beim Gehen, wie in den Figuren 1(a) und 1(b) gezeigt ist, oder im Testbetrieb der Prothese, wirken durch Auftreten des Fußteils 6 die Fig. 2 dargestellten Kräfte bzw. Kraftkomponenten. Zum einen die axiale, in z-Richtung wirkende Kraft Fₐₓ auf den in z-Richtung orientierten Sensorteil 22, zum anderen ein Biegemoment B. Ein Torsionsmoment T bezüglich der z-Achse spielt aufgrund der ringförmigen Ausbildung des Außenteils 16 nahezu keine Rolle, da der ovale Ring torsionssteif ist. Dasselbe gilt für laterale Biegemomente in y-Richtung. Wie aus den Figuren 5(a) und 5(b) ersichtlich ist, führt die axiale Kraft bzw. Kraftkomponente Fₐₓ zu einer Verkürzung und Verbreiterung des ovalen Rings des Außenteils 16 und damit zu einer meßbaren Längenänderung des Sensorteils 22, die mit dem Sensor 31 erfaßt wird. Das auftretende Biegemoment B führt zu einer Verkürzung bzw. Stauchung des einen seitlichen Stegs 18b des Außenteils und zu einer Verlängerung des gegenüberliegenden Stegs 18a, was mit den an den Innenseiten der Stege 18a, 18b vorgesehenen Sensoren 19 erfaßt wird. Durch Diffenzbildung der erfaßten Werte wird das Biegemoment bestimmt. Wie in Fig. 5(b) gezeigt ist, führt das Biegemoment B jedoch zu keiner meßbaren Längenänderung des Sensorteils 22, so daß die axiale Kraft ohne Beeinflussung durch das Biegemoment erfolgt. Aufgrund der beschriebenen Ausbildung des Außenteils 16 ist dieses so stabil, daß darüber die Hauptlast, die auf die Prothese wirkt übertragen wird.

Etwaige doch über die Stege 24 des Innenkörpers 21 übertragenen Biegemomente werden durch die Ausbildung des Innenkörpers als Ring 23 um das Sensorteil 22 abgefangen, da dieser eine Biegesteifigkeit aufweist. Der Ring 23 des Innenkörpers mit den verbreiterten kurzen Seiten wirkt somit als Biegeentlastung für das Sensorteil 22. Die Dicke der verbreiterten Seiten und damit ihre Masse werden in Abhängigkeit von der benötigten Biegeentlastung gewählt.

Mit der oben beschriebenen Sensoreinrichtung ist somit eine Messung der axialen Kraft ohne Beeinflussung durch Biegemomente möglich. Die durch die erfindungsgemäße Sensoreinrichtung erfolgende Lasttrennung erlaubt es, die relativ geringen Verformungen durch die Axialkraft präzise zu messen. Damit lassen sich verschiedene Gangphasen, wie Bodenauftritt, Abrollen und Abheben des Fußteils bestimmen.

Bei der oben beschriebenen Ausführungsform sind Außen- und Innenkörper als zwei Teile beschrieben, die zusammengefügt und fest miteinander verbunden sind. Die Sensoreinrichtung 15 kann aber auch einstückig, z.B. als erodiertes oder gegossenes Teil ausgebildet sein. Umgekehrt muß die Sensoreinrichtung nicht einstückig mit dem Schienbeinteil 4 ausgebildet sein, sondern kann auch über eine geeignete Befestigungsvorrichtung, z.B. Schrauben mit dem Schienbeinteil verbunden sein.

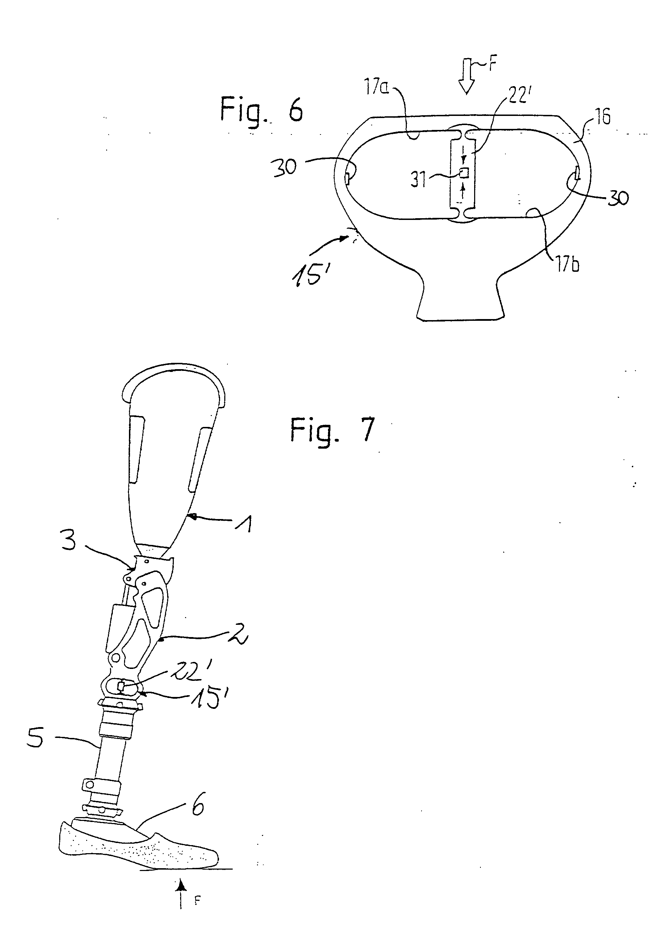

In der in Fig. 6 gezeigten abgewandelten Ausführungsform ist die Sensoreinrichtung 15' ähnlich der Sensoreinrichtung 15, wobei aber der Innenkörper lediglich aus dem Steg 22' gebildet ist, ohne daß ein Ring 23 vorgesehen ist. Dies ist im Prinzip ausreichend, da der Außenkörper 16 bereits das Biegemoment aufnimmt. Eine Beinprothese mit einer solchen Sensoreinrichtung 15' ist in Fig. 7 gezeigt. Das Biegemoment kann über an den Innenseiten der Stege 18a, 18b vorgesehene Sensoren 30 gemessen werden, wie bei der Ausführungsform gemäß Fig. 3.

In einer weiteren abgewandelten Ausführungsform sind die Sensoren 30 an den Innenseiten der Stege 18a, 18b nicht vorhanden, sondern die Biegemomentmessung erfolgt durch separate am Unterschenkelrohr 5 oder unterhalb des Kniegelenks 3 an anderer Stelle als der Sensoreinrichtung 15' im Schienbeinteil 2 vorgesehene Sensoren.

Die Sensoren 30, 31 müssen nicht als DMS-Streifen ausgebildet sein, sondern können piezoelektrische oder Hall-Sensoren oder andere Sensoren sein.

In einer weiteren abgewandelten Ausführungsform ist der ovale Ring des Außenkörpers als Rechteck ausgebildet. Der Ausdruck ringförmig bzw. Ring, so wie er im Zusammenhang mit dieser Erfindung in der Beschreibung und den Ansprüchen sowohl für den Außenkörper als auch für den Innenkörper verwendet ist, ist jedoch nicht auf einen kreisförmigen, ovalen, rechteckigen, oder polygonalen Ring beschränkt sondern umfaßt jede geschlossene Struktur, bei der die Dicke und Breite der Struktur im Verhältnis zu dem umschlossenen Raum so ist, daß sich unter Einwirkung eines Biegemoments die beschriebene Verformung ergibt.

## Patentansprüche

1. Sensoreinrichtung an der Verbindungsstelle zwischen zwei Teilen (4, 5), insbesondere bei einer Prothese, mit
einem ringförmig geschlossen ausgebildeten Außenkörper (16) zum Verbinden der beiden Teile (4, 5) und
einem zwei gegenüberliegende Innenseiten (17a, 17b) des Außenkörpers verbindendem Innenkörper (21, 22, 22') mit einem Sensorelement (31) zur Messung der in Richtung der Verbindungsachse zwischen den beiden Teilen (4, 5) wirkenden Kraft.

2. Sensoreinrichtung nach Anspruch 1, **dadurch gekennzeichnet, dass** ein Innendurchmesser des Außenkörpers (16) an der Stelle des Innenkörpers (21) kleiner ist, als in einer Richtung senkrecht dazu.

3. Sensoreinrichtung nach Anspruch 1 oder 2, **dadurch gekennzeichnet, dass** der Außenkörper an gegenüberliegenden Seiten in einer Richtung senkrecht zur Richtung der Verbindungsachse wenigstens ein Sensorelement (30) zur Erfassung von einer auf den Außenkörper (16) außerhalb der Verbindungsachse des Innenkörpers wirkenden Kraft aufweist.

4. Sensoreinrichtung nach einem der Ansprüche 1 bis 3, **dadurch gekennzeichnet, dass** der Innenkörper (21) eine zusätzlich Einrichtung (23) zur Biegeentlastung des das Sensorelement (31) tragenden Teils (22) des Innenkörpers (21) aufweist.

5. Sensoreinrichtung nach einem der Ansprüche 1 bis 3, **dadurch gekennzeichnet, dass** der Außenkörper (16) einen ovalen Ring bildet und der Innenkörper (22') stegförmig ausgebildet ist und die Längsseiten des ovalen Rings miteinander verbindet.

6. Sensoreinrichtung nach einem der Ansprüche 1 bis 5, **dadurch gekennzeichnet, dass** der Innenkörper (21) als im wesentlichen rechteckiger Ring (23) mit einem Mittelsteg (22) ausgebildet ist, wobei das Sensorelement auf dem Mittelsteg (22) vorgesehen ist.

7. Sensoreinrichtung nach einem der Ansprüche 1 bis 6, **dadurch gekennzeichnet, dass** der Querschnitt des Innenkörpers (21) an der Stelle des Sensorelements (31) kleiner ist, als der Querschnitt des Außenkörpers an der Stelle der Krafteinwirkung in Richtung der Verbindungsachse.

8. Sensoreinrichtung nach einem der Ansprüche 1 bis 7, **dadurch gekennzeichnet, dass** an dem Außenkörper (16) symmetrische zu beiden Seiten des Innenkörpers Sensorelemente (30) zur Bestimmung des auf den Außenkörper wirkenden Biegemoments vorgesehen sind.

9. Sensoreinrichtung nach einem der Ansprüche 1 bis 8, **dadurch gekennzeichnet, dass** der Außenkörper und der Innenkörper miteinander fest verbunden sind.

10. Sensoreinrichtung nach einem der Ansprüche 1 bis 9, **dadurch gekennzeichnet, dass** der Außenkörper und der Innenkörper aus demselben Material gebildet sind.

11. Sensoreinrichtung nach einem der Ansprüche 1 bis 10, **dadurch gekennzeichnet, dass** die Sensoreinrichtung (15) an dem Unterschenkelteil (2) einer Beinprothese vorgesehen ist.

12. Sensoreinrichtung nach einem der Ansprüche 1 bis 10, **dadurch gekennzeichnet, dass** die Sensoreinrichtung eine Sensoreinrichtung für eine Prothese, bevorzugt eine Beinprothese ist.

13. Beinprothese mit einem Oberschenkelteil (1), einem Unterschenkelteil (2) und einem künstlichen Kniegelenk (3), **dadurch gekennzeichnet, dass** eine Sensoreinrichtung nach einem der Ansprüche 1 bis 10 vorgesehen ist.

14. Beinprothese nach Anspruch 13, **dadurch gekennzeichnet, dass** das Unterschenkelteil angrenzend an das künstliche Kniegelenk ein Schienbeinteil (4) aufweist und die Sensoreinrichtung (15) in das Schienbeinteil integriert ist.

15. Sensoreinrichtung zum Messen von mechanischen Kräften mit einem Sensorabschnitt (22) mit einem Kraftsensor (31) zum Messen von einer auf den Sensorabschnitt (22) in einer ersten Richtung wirkenden Kraft (Fₐₓ) und einem mit dem Sensorabschnitt (22) verbundenen Biegeentlastungsabschnitt (16, 23), der derart ausgebildet ist, dass er sich unter Einwirkung einer in einer zweiten Richtung wirkenden Kraft so verformt, dass der Sensorabschnitt (22) im wesentlichen unbeeinflusst bleibt.

16. Sensoreinrichtung nach Anspruch 15, **dadurch gekennzeichnet, dass** der Biegeentlastungsabschnitt (16, 23) derart ausgebildet ist, dass er ein zusätzlich zu der in der ersten Richtung wirkenden Kraft (Fₐₓ) wirkendes Biegemoment (B) aufnimmt, wobei der Sensorabschnitt (22) im wesentlichen unbeeinflusst bleibt.

17. Sensoreinrichtung nach Anspruch 15 oder 16, **dadurch gekennzeichnet, dass** der Biegeentlastungsabschnitt (16, 21) wenigstens einen Sensor (30) zur Messung des Biegemoments (B) aufweist.

18. Sensoreinrichtung nach einem der Ansprüche 15 bis 17, **dadurch gekennzeichnet, dass** der Sensorabschnitt (22) als Innenkörper und der Biegeentlastungsabschnitt als Außenkörper gemäß einem der Ansprüche 1 bis 12 ausgebildet ist.
